# EUROPÄISCHE PATENTSCHRIFT

(11) **EP 1 004 566 B1**
(45) Veröffentlichungstag und Bekanntmachung des Hinweises auf die Patenterteilung: **18.09.2002**
(21) Anmeldenummer: 99121203.6
(22) Anmeldetag: 23.10.1999
(51) Int. Cl.: C07C 45/33, B01J 31/18

(54) **Verfahren zur Oxidation von Methyl-, Methylen- oder Methingruppen enthaltenden Substraten**
Process for oxidation of substrates containing methyl, methylene or methin groups
Procédé d'oxydation de substrats comprenant des groupes de méthyle, méthylène ou méthine

(30) Priorität: 25.11.1998 AT 197498; 25.11.1998 AT 197598; 29.06.1999 AT 112799
(43) Veröffentlichungstag der Anmeldung: 31.05.2000
(73) Patentinhaber: DSM Fine Chemicals Austria Nfg GmbH & Co KG, 4021 Linz (AT)
(72) Erfinder: Alsters, Paul, 6224 KZ Maastricht (NL); Bouttemy, Sabine, 6165 Geleen (NL)
(74) Vertreter: Klostermann, Ingrid

(56) Entgegenhaltungen:
- EP-A- 0 198 351
- EP-A- 0 824 962
- EINHORN C ET AL: "OXIDATION OF ORGANIC SUBSTRATES BY MOLECULAR OXYGEN MEDIATED BY N-HYDROXYPHTHALIMIDE (NHPI) AND ACETALDEHYDE" CHEMICAL COMMUNICATIONS,GB,ROYAL SOCIETY OF CHEMISTRY, Nr. 5, 7. März 1997 (1997-03-07), Seiten 447-448, XP002072174 ISSN: 1359-7345
- DATABASE WPI Section Ch, Week 199937 Derwent Publications Ltd., London, GB; Class E13, AN 1999-439414 XP002138092 & JP 11 180913 A (DAICEL CHEM IND LTD), 6. Juli 1999 (1999-07-06)

## Beschreibung

Die Oxidationsreaktion gehört zu den grundlegendsten Reaktionen in der organischen Chemie, sodaß bereits eine Vielzahl von Varianten in der Literatur beschieben ist. Als geeigneter Katalysator für Oxidationsreaktionen unter milden Bedingungen wurde in letzter Zeit N-Hydroxyphthalimid (NHPI) bekannt.
Gemäß EP-A1-0 824 962 wird NHPI bzw. andere Imidverbindungen mit einem Metallcokatalysator kombiniert und zur Oxidation einer Vielzahl von organischen Substanzen eingesetzt. Die Oxidation von Isoprenoiden, die eine allylische Gruppe besitzen, zu Hydroperoxiden, die anschließend in die entsprechenden Alkohole, Aldehyde oder Ketone umgewandelt werden, ist in EP 0 198 351 beschrieben. Dabei werden N-Hydroxydicarbonsäureimide als Katalysator verwendet.
Als Variante beschreibt Einhorn in Chem. Commun. 1997, S. 447-448 die Verwendung von NHPI in Kombination mit Acetaldehyd als Katalysatoren für die Oxidation von organischen Substraten, insbesonders von Kohlenwasserstoffen.

Trotzdem fehlt es an Oxidationssystemen mit Sauerstoff als Oxidator, die eine selektive Oxidation unter möglichst milden Bedingungen gewährleisten, da die bisher bekannten Varianten bei vielen Verbindungen eine geringe Selektivität und niedrige Reaktionsgeschwindigkeit als Nachteile aufweisen.

Unerwarteterweise wurde nun gefunden, daß die Verwendung einer Kombination, bestehend aus Imidverbindung und Metallcokatalysator, in Gegenwart eines Aldehyds als Cosubstrat die Oxidation einer Vielzahl von organischen Substraten unter äußerst milden Bedingungen mit hoher Selektivität und hoher Reaktionsgeschwindigkeit ermöglicht.

Gegenstand der vorliegenden Erfindung ist demnach ein Verfahren zur Oxidation von Methyl-(CH₃), Methylen-(CH₂) oder Methin-(CH)gruppen enthaltenden Substraten mittels Sauerstoffoxidation unter Verwendung eines Katalysatorsystemes, aus einer Imidverbindung und einem Metallcokatalysator, das dadurch gekennzeichnet ist, daß die Substrate mit einem aromatischen oder aliphatischen Aldehyd mit 2 - 20 C-Atomen, in Gegenwart des Katalysatorsystemes bestehend aus
a) einer Imidverbindung der Formel 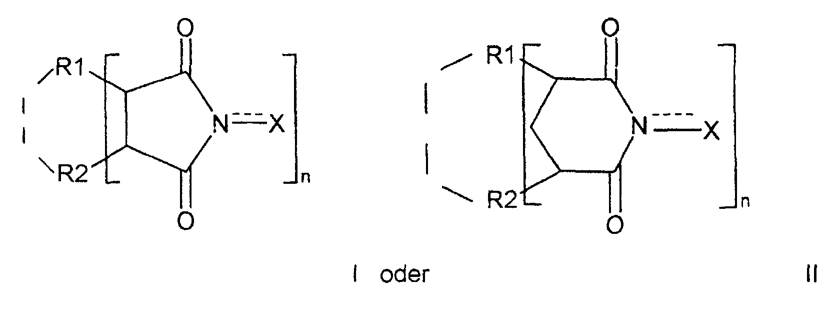 in denen R₁ und R₂ H, OH, Halogen, eine C₁-C₂₀-Alkyl-, Alkenyl- oder Alkoxygruppe, eine Arylgruppe, eine C₁-C₆-Acylgruppe, eine Carboxyl- oder eine C₁-C₁₀-Alkoxycarbonylgruppe bedeuten können, oder R₁ und R₂, in der Formel I gemeinsam eine Doppelbindung oder, in der Formel I oder II ein aromatisches oder nicht-aromatisches Ringsystem bilden, X O oder OH bedeuten und n eine ganze Zahl von 1 bis 3 sein kann, und
b) einem Cokatalysator enthaltend ein oder mehrere Elemente aus der Gruppe der Übergangsmetalle, der 2A und 3A Elemente des Periodensystems,
in einem organischen Lösungsmittel zu den korrespondierenden Oxoverbindungen oxidiert werden.

Als Ausgangsverbindung für das erfindungsgemäße Verfahren dienen Methyl- Methylen-, oder Methingruppen enthaltende Substrate. Geeignete Subtrate sind beispielsweise in EP-A1-0 824 962 beschrieben und beinhalten aliphatische, alicyclische oder aromatische Kohlenwasserstoffe, die gesättigt oder ein- oder mehrfach ungesättigt sein können, Heterocyclen, Alkohole, Ester, Aldehyde, Ketone und Amine. Die Substrate können dabei einen oder mehrere Substituenten, wie etwa Halogene (F, Cl, Br J), C₁-C₆-Alkylgruppen, Oxogruppen, Hydroxylgruppen, C₁-C₆-Alkoxygruppen, Hydroxy, C₁-C₄-Alkylgruppen, Carboxylgruppen, C₁-C₆-Alkoxycarbonylgruppen, C₁-C₆-Acylgruppen, Aminogruppen, substituierte Aminogruppen, Cyanogruppen, Nitrogruppen und dergleichen aufweisen.

Bevorzugt werden mit dem erfindungsgemäßen Verfahren Verbindungen, welche eine Aryl-CH₂-Gruppe enthalten, wie etwa Indane, 1,2-Diphenylethan und Dibenz(b,f)-azepine, z. B. 10,11-Dihydro-5H-dibenz(b,f)-azepincarboxamid, oder Verbindungen, die durch Carboxyl- oder Carbonylgruppen aktivierte Methylengruppen enthalten, oxidiert.

Das erfindungsgemäße Verfahren verwendet ein Katalysatorsystem, bestehend aus 2 Komponenten, in Kombination mit einem Aldehyd als Cosubstrat.
Ein geeignetes Katalysatorsystem ist beispielsweise in EP 0 824 962 beschrieben. Geeignete Imidverbindungen der Formel I für Komponente a) sind demnach Verbindungen der Formel I in der R₁ und R₂ somit gleich oder verschieden sein können und Wasserstoff, Hydroxy, Halogen wie J, Cl, F, Br, C₁ - C₁₀ -Alkyl oder Alkoxy bedeuten.
Unter C₁ - C₂₀ Alkyl sind geradkettige, verzweigte oder cyclische Alkylreste wie etwa Methyl, Ethyl, Propyl, Isopropyl, Butyl, Isobutyl, sec. Butyl, t-Butyl, Pentyl, Cyclopentyl, Hexyl, Cyclohexyl, Heptyl, Octyl, Cyclooctyl, Decyl, Dodecyl u.s.w. zu verstehen.
Bevorzugt sind C₁ bis C₁₅ Alkylgruppen, besonders bevorzugt C₁ bis C₁₂ -Alkylgruppen.
Unter C₁ - C₂₀-Alkenyl sind geradkettige oder verzweigte Alkenylreste zu verstehen.
Beispiele dafür sind Propenyl, Hexenyl, Octenyl, Decenyl, Dodecenyl u.s.w.
Bevorzugt sind C₄ bis C₁₈ -Alkenylgruppen, besonders bevorzugt C₆ bis C₁₂ Alkenylgruppen.
Unter C₁ - C₂₀ -Alkoxygruppen sind beispielsweise Methoxy-, Ethoxy-, Propoxy-, Isopropoxy-, Butoxy-, Isobutoxy-, t-Butoxy-, Pentyloxy-, Hexyloxygruppen u.s.w. zu verstehen. Bevorzugt sind wiederum C₁ - C₆, besonders bevorzugt C₁ - C₄ - Alkoxygruppen.
Weiters können R₁ und R₂ eine Arylgruppe, wie etwa eine Phenylgruppe oder eine Naphthylgruppe, eine C₁ - C₆-Acylgruppe, wie etwa Formyl, Acetyl, Propionyl, Butyryl, Isobutyryl, Valeryl, Isovaleryl, Pivaloyl, u.s.w. bedeuten.
R₁ und R₂ können auch eine Carboxylgruppe oder eine Alkoxycarbonylgruppe sein. Geeignete Alkoxycarbonylgruppen sind solche, die 1 - 10 C-Atome im Alkoxyteil enthalten. Beispiele dafür sind Methoxycarbonyl, Ethoxycarbonyl, Propoxycarbonyl, Isopropoxycarbonyl, Butoxycarbonyl, Isobutoxycarbonyl, t-Butoxycarbonyl, Pentyloxycarbonyl, Hexyloxycarbonyl u.s.w. Bevorzugt sind solche Gruppen, die 1 - 6 C-Atome, besonders bevorzugt 1 - 4 C-Atome, im Alkoxyteil enthalten.

R₁ und R₂ können aber auch in der Formel I gemeinsam eine Doppelbindung oder in der Formel I oder II ein aromatisches oder ein nicht aromatisches Ringsystem bilden, das aus einem oder mehreren kondensierten Ringen besteht. Bevorzugt sind aromatische oder nicht aromatische Ringsysteme mit 5 - 12 C-Atomen, bevorzugt mit 6 - 10 C-Atomen. Die Ringe können dabei auch Heterocyclen sein. Beispiele dafür sind Cyclohexan oder andere Cycloalkanringe, die gegebenenfalls substituiert sein können, sowie Cyclohexen oder andere Cycloalkenringe, die wiederum gegebenenfalls substituiert sein können, nicht aromatische, überbrückte Ringe, Benzolring, Naphthalenring und andere gegebenenfalls substituierte aromatische Ringe.
Verbindungen der Formel II können entweder einen gesättigten oder einen ungesättigten N-hältigen 6-Ring aufweisen.
X bedeutet in der Formel I oder II O oder OH.
In Abhängigkeit von der Bedeutung von X, bedeutet die Bindung zwischen N und X entweder eine Doppel- oder eine Einfachbindung. n bedeutet eine ganze Zahl von 1 bis 3, bevorzugt 1 oder 2.

Bevorzugte Imidverbindungen sind N-Hydroxysuccinimid, N-Hydroxymaleimid, N-Hydroxyhexahydrophthalimid, N,N'-Dihydroxycyclohexantetracarboximid, N-Hydroxyphthalimid, N-Hydroxy-tetrabromophthalimid, N-Hydroxytetrachlorophthalimid, N-Hydroxytrimellitimid, N,N'-Dihydroxynaphthalentetracarboximid, N-Hydroxy(dodecenyl)succinimid, N-Hydroxy(octenyl)succinimid, N-Hydroxynaphthalimid u.s.w.

Als Komponente b) wird ein Cokatalysator eingesetzt. Für das erfindungsgemäße Katalysatorsystem eignen sich die in EP 0 824 962 beschriebenen Cokatalysatoren. Der Cokatalysator enthält demnach ein oder mehrere Elemente aus der Gruppe der 2A Elemente des Periodensystems, wie Mg, Ca, Sr, Ba oder der 3A Elemente, wie B oder Al oder der Übergangselemente.
Als Übergangselemente sind beispielsweise Elemente des Periodensystems aus der Gruppe 3B, wie Sc, Y, La, Ce, Sm oder Lanthanoiden, Ac oder andere Actinoiden, aus der Gruppe 4B (Ti, Zr, Hf), Gruppe 5B (V, Nb, Ta), Gruppe 6B (Cr, Mo, W), Gruppe 7B (Mn, Tc, Re), Gruppe 8 (Fe, Ru, Os, Co, Rh, Ir, Ni, Pd, Pt), Gruppe 1B (Cu, Ag, Au) und aus der Gruppe 2B (z.B. Zn, Cd) geeignet.
Bevorzugt werden Cokatalysatoren eingesetzt, die Ti, Zr oder andere Gruppe 4B Elemente, V oder andere 5B Elemente, Cr, Mo, W oder andere Gruppe 6B Elemente, Mn, Tc, Re oder andere Gruppe 7B Elemente, Fe, Ru, Co, Rh, Ni oder andere Gruppe 8 Elemente oder Cu oder andere Gruppe 1B Elemente enthalten.

Besonders bevorzugt sind Kombinationen aus Elementen der Gruppe 6B und/oder 8 oder 1B.
Der Cokatalysator kann dabei als Metallhydroxid, Metalloxid, als organisches Salz, anorganisches Salz, u.s.w. eingesetzt werden.
Hydroxyde sind beispielsweise Mn(OH)₂, MnO(OH), Fe(OH)₂ und Fe(OH)₃. Geeignete Oxide sind beispielsweise Sm₂O₃, TiO₂, ZrO₂, V₂O₃, V₂O₅, CrO, Cr₂O₃, MoO₃, MnO, Mn₃O₄, Mn₂O₃, MnO₂, Mn₂O₇, FeO, Fe₂O₃, Fe₃O₄, RuO₂, RuO₄, CoO, CoO₂, Co₂O₃, Cu₂O₃, u.s.w.
Organische Salze sind beispielsweise Formiate, Acetate, Propionate, Acetylacetonate, Naphthenate, Stearate und andere Salze mit C₂-C₂₀ Fettsäuren von Co, Mn, Ce, Ti, Zr, V, Cr, Mo, Fe, Ru, Ni, Pd, Cu und Zn.
Anorganische Salze beinhalten beispielsweise Nitrate, Sulfate, Phosphate, Carbonate und Halogenide von Co, Fe, Mn, Ni, Cu, u.s.w.
Der Cokatalysator kann auch in Form eines Komplexes, wie etwa in EP-A1-0 824 962 beschrieben, eingesetzt werden.
Bevorzugt wird der Cokatalysator als organisches Salz, z.B. Acetat, Acetylacetonat, u.s.w., sowie als anorganisches Salz, z.B. Nitrat u.s.w. eingesetzt.

Besonders bevorzugt wird als Cokatalysator Ni oder Cu, beispielsweise als Acetat oder Nitrat verwendet.
In einer weiteren besonders bevorzugten Ausführungsform wird Ni oder Cu in Kombination mit Cr als Komponente b) zugesetzt.
Ganz besonders bevorzugt wird die Kombination aus Ni oder Cu mit Cr und Spuren von Co als Cokatalysator eingesetzt.
Die Komponenten a) und b) des erfindungsgemäßen Systems können dabei sowohl ein homogenes als auch ein heterogenes System bilden.
Die Komponenten a) und b) können gewünschtenfalls in fester Form auf einen Träger aufgebracht sein. Geeignete Träger sind beispielsweise Silica, Zeolith, Aktivkohle, u.s.w. oder andere poröse Träger.

Als Cosubstrat wird bei dem erfindungsgemäßen Verfahren ein Aldehyd verwendet. Als Aldehyde eignen sich sowohl gesättigte und ungesättigte aliphatische, wie auch aromatische Aldehyde oder Dialdehyde mit 2 bis 20 C-Atomen. Beispiele dafür sind Acetaldehyd, Propionaldehyd, Butyraldehyd, Valeraldehyd, Capronaldehyd, Benzaldehyd, Naphthaldehyd, o-Phthalaldehyd u.s.w.

Bevorzugt wird als Cosubstrat Acetaldehyd oder Benzaldehyd eingesetzt.

Die eingesetzten Mengen der einzelnen Komponenten betragen
für Komponente a):
   0,001 bis 0,7 mol, besonders bevorzugt 0,01 bis 0,5 mol pro mol Substrat
für Komponente b):
   0,0001 mol bis 0,5 mol, bevorzugt 0,0001 bis 0,3 mol, besonders bevorzugt 0,001 bis 0,2 mol pro mol Substrat und
für das Cosubstrat:
   0,1 bis 3 mol, bevorzugt 0,2 bis 2,5 mol pro mol Substrat

Das molare Verhältnis von Cokatalysator zu Imidverbindung liegt bei 0,001 bis 1 mol, bevorzugt bei 0,001 bis 0,8 mol, besonders bevorzugt bei 0,002 bis 0,2 mol pro mol Imidverbindung.

Wird als Cokatalysator eine Kombination aus Ni oder Cu und Cr eingesetzt, so wird Cr nur in Spuren im Vergleich zur Ni- bzw. Cu-Menge eingesetzt. Bevorzugt wird demnach 0,1 bis 0,25 mol Cr pro mol Ni oder Cu verwendet.
Bei Verwendung der ganz besonders bevorzugten Kombination aus Ni oder Cu mit Spuren von Cr und Co wird Co in einer Menge von 0,0001 bis 0,003 mol pro mol Ni oder Cu eingesetzt.

Das erfindungsgemäße Verfahren wird in einem organischen Lösungsmittel durchgeführt. Als Lösungsmittel eignen sich Ameisensäure, Essigsäure, Propionsäure und andere Carbonsäuren oder Hydroxycarbonsäuren, Acetonitril, Propionnitril, Benzonitril und andere Nitrile, Formaldehyd, Acetamid, Dimethylformamid, Dimethylacetamid und andere Amide, t-Butanol, t-Amylalkohol und andere Alkohole, Hexan, Octan und andere aliphatische Kohlenwasserstoffe, Benzol, Chloroform, Dichlormethan, Dichlorethan, Tetrachlorkohlenstoff, Nitrobenzol, Nitromethan, Ethylacetat, Butylacetat, Dimethylether, Diethylether, Diisopropylether sowie Mischungen dieser Lösungsmittel. Bevorzugt werden Essigsäure, Propionsäure, Acetonitril, Benzonitril, Chloroform oder Dichlormethan als Lösungsmittel verwendet. Gegebenenfalls kann auch das Substrat selbst als Lösungsmittel, bei Verwendung im Überschuß, dienen. Als Oxidationsmittel dient Sauerstoff im Überschuß, der in Form von reinem Sauerstoff oder als Luft zugeführt werden kann. Weiters kann der Sauerstoff auch durch ein Inertgas, wie etwa Stickstoff, Helium, Argon oder Kohlendioxid verdünnt werden.

Die Reaktionstemperatur ist vom eingesetzten Substrat abhängig und kann zwischen -20 und 100°C liegen, bevorzugt wird die Oxidation bei Raumtemperatur von 0 bis 30°C durchgeführt.
Die Reaktion kann bei Normaldruck oder bei erhöhtem Druck durchgeführt werden. Bei dem erfindungsgemäßen Verfahren wird das zu oxidierende Substrat in einem der oben angeführten Lösungsmittel gelöst und das Katalysatorsystem, bestehend aus den Komponenten a) und b) zugegeben.

Der Aldehyd kann zu Beginn der Reaktion dem Reaktionsgemisch in einer Portion zugegeben werden, oder während der Reaktion über einen längeren Zeitraum zudosiert werden. Es ist auch möglich, einen Teil des Aldehyds zu Beginn der Reaktion vorzulegen und den Rest während der Reaktion zuzudosieren.

Das erfindungsgemäße Verfahren kann, in Abhängigkeit vom eingesetzten Substrat, zur Herstellung einer Vielzahl von Oxoverbindungen verwendet werden. Herstellbare Oxoverbindungen sind Säuren, Alkohole, Ketone, Aldehyde oder Peroxide.
Nach beendeter Reaktion kann das gewünschte Endprodukt auf einfache Weise mittels bekannter Technologien, wie Filtration, Fällung, Kondensation, Destillation, Extraktion, Kristallisation, Chromatographie und dergleichen aus dem Reaktionsgemisch isoliert und gegebenenfalls gereinigt werden.

Die entsprechenden Oxoverbindungen werden durch das erfindungsgemäße Verfahren im Vergleich zum Stand der Technik in höherer Selektivität und Ausbeute sowie Reaktionsgeschwindigkeit erhalten.

Das in den Beispielen 1 - 3 verwendete Ni(OAc)₂.4H₂O hat eine Reinheit von 98% (Aldrich) und enthält etwa 100 ppm Co.
Das in Beispiel 4 verwendete Ni(OAc)₂.4H₂O ist 100% rein (Baker).

### Beispiel 1:

0,239 g (1,00 mmol) IDB-CONH₂ wurden in 3 ml Acetonitril gelöst. Es wurden 0,061 g (0.37 mmol) N-Hydroxyphthalimid, 0,0022 g (0.0088 mmol) Nickelacetat und 0,001 g (0.0025 mmol) Chrom(III)nitrat zugegeben. Als Cosubstrat wurden 0,1185 g (1.12 mmol) Benzaldehyd in einer Portion zugesetzt.
Die Oxidation erfolgte bei 1 bar Sauerstoff und Raumtemperatur. Nach einer Reaktionszeit von 17 Stunden wurde das Reaktionsgemisch mittels HPLC analysiert. Es wurden 36% Oxcarbazepin bei 45% Umsatz (= 81% Selektivität bezogen auf Oxcarbazepin) erhalten.

### Beispiel 2:

Beispiel 2 wurde analog Beispiel 1 durchgeführt. Anstelle von Acetonitril wurde Essigsäure als Lösungsmittel verwendet.
Es wurden 49% Oxcarbazepin bei 75% Umsatz (= 66% Selektivität bezogen auf Oxcarbazepin) erhalten.

### Beispiel 3:

2,38 g (10,0 mmol) IDB-CONH₂ wurden in 30 ml Essigsäure gelöst. Es wurden 0,66 g (4,0 mmol) N-Hydroxyphthalimid, 0,0245 g (0,098 mmol) Nickelacetat und 0,0107 g (0,027 mmol) Chrom(III)nitrat zugegeben. Als Cosubstrat wurden 0,3176 g (3 mmol) Benzaldehyd in einer Portion zugesetzt. Innerhalb von 5 Stunden wurde ein Gemisch von 1,096 g (9,2 mmol) Benzaldehyd und 0,1 ml Essigsäure zugegeben.
Die Oxidation erfolgte bei 1 bar Sauerstoff und Raumtemperatur. Nach einer Reaktionszeit von 7 Stunden wurde das Reaktionsgemisch mittels HPLC analysiert. Es wurden 53% Oxcarbazepin bei 72% Umsatz (= 74% Selektivität bezogen auf Oxcarbazepin) erhalten.

### Vergleichsversuch 1:

Analog zu EP 0 824 962 wurde 0.239 g (1.00 mmol) IDB-CONH₂ in Essigsäure bei Raumtemperatur und 1 bar Sauerstoff in Gegenwart von 0.057 g (0.35 mmol) N-Hydroxyphthalimid und 0.0063 g (0.0253 mmol) Cobaltacetat als Katalysatorsystem, jedoch ohne Cosubstrat oxidiert. Es wurden 16% Oxcarbazepin bei 33% Umsatz (= 48% Selektivität) erhalten.

### Vergleichsversuch 2:

0,245 g (1,03 mmol) IDB-CONH₂ wurden in 3 ml Acetonitril gelöst. Es wurden 0,032 g (0,2 mmol) N-Hydroxyphthalimid, jedoch kein Metallsalz-hältiger Cokatalysator zugesezt. Als Cosubstrat wurde eine Lösung von 0,234 g (5,3 mmol) Acetaldehyd in 2,1 g Acetonitril innerhalb 180 Minuten zugegeben.
Die Oxidation erfolgte bei 1 bar Sauerstoff und Raumtemperatur. Nach einer Reaktionszeit von 17 Stunden wurde das Reaktionsgemisch mittels HPLC analysiert. Es wurden 19% Oxcarbazepin bei 42% Umsatz (= 45% Selektivität bezogen auf Oxcarbazepin) erhalten. Dieses Ergebnis war schlecht reproduzierbar.

### Beispiel 4:

### Oxidation von IDB-CONH₂ mit unterschiedlichen Mengen an Cobaltzusatz

Eine Mischung von 2.38 g (10 mmol) IDB-CONH₂, 0.66 g (4.0 mmol) N-Hydroxyphthalimid (NHPI), 24.9 mg (0.1 mmol) Ni(OAc)₂.4H₂O und 8.8 mg (25 µmol) Chromacetat wurde in Co(OAc)₂.4H₂O enthaltender AcOH gelöst (31 g AcOH). Anschließend wurde bei 22 °C ein langsamer Sauerstoffstrom durch das gerührte Reaktionsgemisch geblasen. 306 mg (2.9 mmol) Benzaldehyd wurden zugesetzt. Nachfolgend wurde ein Gemisch aus 1.14g (10.7 mmol) Benzaldehyd und 0.11g Essigsäure innerhalb von 300 Minuten zugegeben. Das Reaktionsgemisch wurde mittels HPLC nach 300 und nach 420 Minuten analysiert.
Die eingesetzte Co-Menge, die Konversion und Ausbeute sind aus Tabelle 1 ersichtlich. Weiters ist die Zeit angegeben, nach der das Gemisch eine gelbe Farbe erhielt.

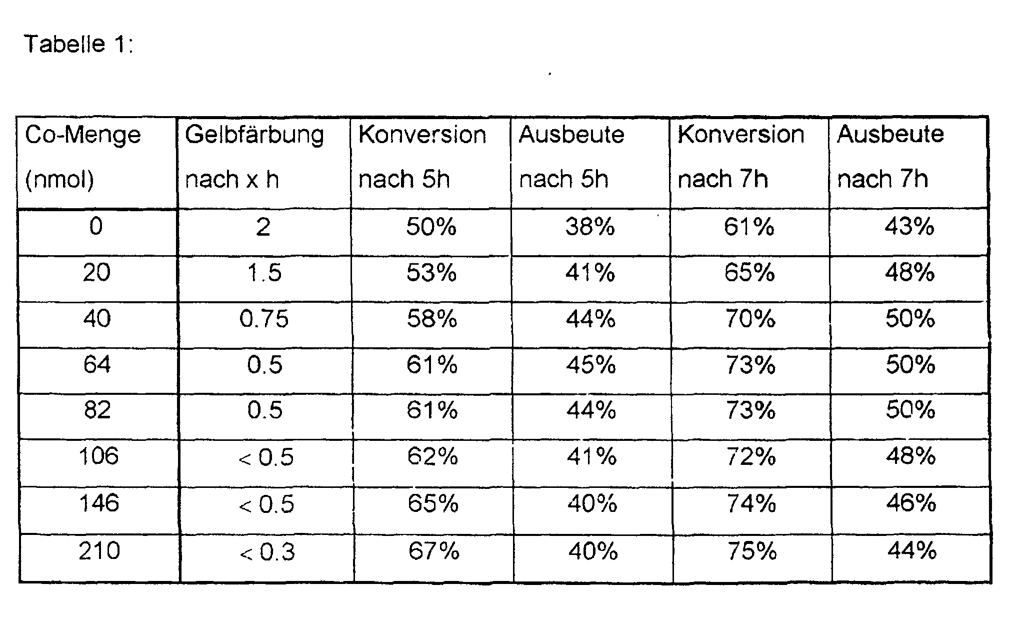

## Patentansprüche

1. Verfahren zur Oxidation von Methyl (CH₃)-, Methylen (CH₂)- oder Methin (CH)-gruppen enthaltenden Substraten mittels Sauerstoffoxidation unter Verwendung eines Katalysatorsystemes aus einer Imidverbindung und einem Metallcokatalysator, **dadurch gekennzeichnet, daß** die Substrate mit einem aromatischen oder aliphatischen Aldehyd mit 2 - 20 C-Atomen, in Gegenwart des Katalysatorsystemes bestehend aus
a) einer Imidverbindung der Formel 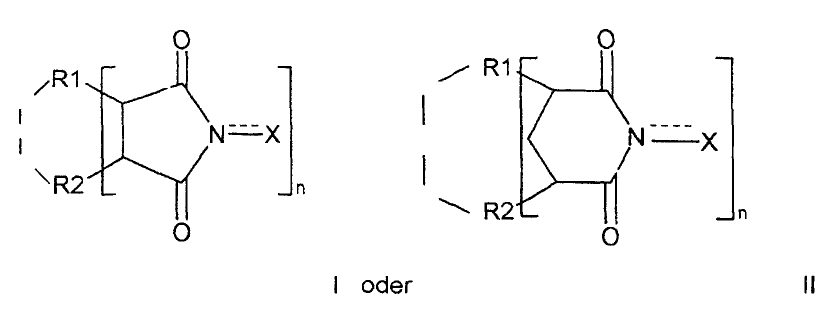 in denen R₁ und R₂ H, OH, Halogen, eine C₁-C₂₀-Alkyl-, Alkenyl- oder Alkoxygruppe, eine Arylgruppe, eine C₁-C₆-Acylgruppe, eine Carboxyl- oder eine C₁-C₁₀-Alkoxycarbonylgruppe bedeuten können, oder R₁ und R₂, in der Formel I gemeinsam eine Doppelbindung oder, in der Formel I und II ein aromatisches oder nicht-aromatisches Ringsystem bilden, X O oder OH bedeutet und n eine ganze Zahl von 1 bis 3 sein kann,
b) einem Cokatalysator enthaltend ein oder mehrere Elemente aus der Gruppe der Übergangsmetalle und der 2A und 3A Elemente des Periodensystems in einem organischen Lösungsmittel mittels Sauerstoff zu den korrespondierenden Oxoverbindungen oxidiert werden.

2. Verfahren nach Anspruch 1, **dadurch gekennzeichnet, daß** als Komponente a) des Katalysatorsystemes N-Hydroxysuccinimid, N-Hydroxymaleimid, N-Hydroxyhexahydrophthalimid, N,N'-Dihydroxycyclohexantetracarboximid, N-Hydroxyphthalimid, N-Hydroxy-tetrabromophthalimid, N-Hydroxytetra-chloro-phthalimid, N-Hydroxytrimellitimid, N,N'-Dihydroxynaphthalentetra-carboximid, N- Hydroxy-(dodecenyl)succinimid, N- Hydroxy(octenyl)succinimid oder N-Hydroxynaphthalimid eingesetzt wird.

3. Verfahren nach Anspruch 1, **dadurch gekennzeichnet, daß** Cokatalysatoren eingesetzt werden, die ein oder mehrere Elemente aus den Gruppen 1B, 4B, 5B, 6B,7B oder 8 des Periodensystemes enthalten.

4. Verfahren nach Anspruch 3, **dadurch gekennzeichnet, daß** ein Cokatalysator eingesetzt wird, der eine Kombination aus Elementen der Gruppe 6B und 8 oder 1B enthält.

5. Verfahren nach Anspruch 1, **dadurch gekennzeichnet, daß** der Cokatalysator als Formiat, Acetat, Propionat, Acetylacetonat, Naphthenat, Stearat, Nitrat, Sulfat, Phosphat, Carbonat oder Halogenid eingesetzt wird.

6. Verfahren nach Anspruch 1, **dadurch gekennzeichnet, daß** als Cosubstrat ein gesättigter oder ungesättigter aliphatischer oder aromatischer Aldehyd oder Dialdehyd mit 2-20 C-Atomen eingesetzt wird.

7. Verfahren nach Anspruch 6, **dadurch gekennzeichnet, daß** als Aldehyd Acetaldehyd, Propionaldehyd, Butyraldehyd, Valeraldehyd, Capronaldehyd, Benzaldehyd oder o-Phthalaldehyd eingesetzt wird.

8. Verfahren nach Anspruch 1, **dadurch gekennzeichnet, daß** als Substrat Verbindungen, welche eine Aryl-CHₙ- (n= 1, 2 oder 3) Gruppe enthalten oder Verbindungen, die durch Carboxyl- oder Carbonylgruppen aktivierte Methylen- oder Methingruppen enthalten, eingesetzt werden.

9. Verfahren nach Anspruch 1, **dadurch gekennzeichnet, daß** pro mol Substrat 0,001 bis 0,7 mol an Komponente a), 0,0001 mol bis 0,5 mol an Komponente b) und 0,1 bis 3 mol an Cosubstrat eingesetzt werden.

10. Verfahren nach Anspruch 1, **dadurch gekennzeichnet, daß** als Lösungsmittel Ameisensäure, Essigsäure, Propionsäure, Acetonitril, Propionnitril, Benzonitril, Formaldehyd, Acetamid, Dimethylformamid, Dimethylacetamid, t-Butanol, t-Amylalkohol, Hexan, Octan, Benzol, Chloroform, Dichlormethan, Dichlorethan, Tetrachlorkohlenstoff, Nitrobenzol, Nitromethan, Ethylacetat, Butylacetat, Dimethylether, Diethylether, Diisopropylether oder Mischungen davon, eingesetzt werden.

## Claims

1. Process for the oxidation of substrates containing methyl (CH₃), methylene (CH₂) or methine (CH) groups by means of oxygen oxidation with the use of a catalyst system comprising an imide compound and a metal cocatalyst, **characterized in that** substrates are oxidized by means of oxygen together with an aromatic or aliphatic aldehyde having 2-20 C atoms in the presence of a catalyst system consisting of
a) an imide compound of the formula 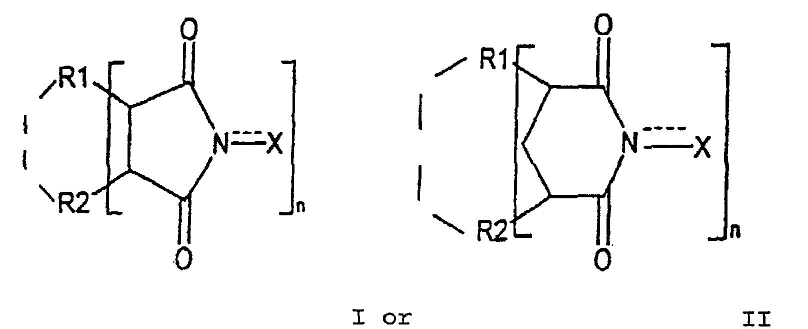 in which R₁ and R₂ may denote H, OH, halogen, a C₁-C₂₀-alkyl, alkenyl or alkoxy group, an aryl group, a C₁-C₆-acyl group, a carboxyl or a C₁-C₁₀-alkoxycarbonyl group, or R₁ and R₂ together form a double bond in the formula I or form an aromatic or non-aromatic ring system in the formula I or II, X may denote O or OH and n may be an integer from 1 to 3, and
b) a cocatalyst containing one or more elements from the group consisting of the transition metals and
of the 2A and 3A elements of the Periodic Table, in an organic solvent to give the corresponding oxo compounds.

2. Process according to Claim 1, **characterized in that** N-hydroxysuccinimide, N-hydroxymaleimide, N-hydroxyhexahydrophthalimide, N,N'-dihydrocyclohexanetetracarboximide, N-hydroxyphthalimide, N-hydroxytetrabromophthalimide, N-hydroxytetrachlorophthalimide, N-hydroxytrimellitimide, N,N'-dihydroxynaphthalenetetracarboximide, N-hydroxy(dodecenyl)succinimide, N-hydroxy(octenyl)succinimide or N-hydroxynaphthalimide is used as component a) of the catalyst system.

3. Process according to Claim 1, **characterized in that** cocatalysts which contain one or more elements of groups 1B, 4B, 5B, 6B, 7B or 8 of the Periodic Table are used.

4. Process according to Claim 3, **characterized in that** a cocatalyst which contains a combination of elements of group 6B and 8 or 1B is used.

5. Process according to Claim 1, **characterized in that** the cocatalyst is used in the form of a formate, acetate, propionate, acetylacetonate, naphthenate, stearate, nitrate, sulphate, phosphate, carbonate or halide.

6. Process according to Claim 1, **characterized in that** a saturated or unsaturated aliphatic or aromatic aldehyde or dialdehyde having 2-20 C atoms is used as the cosubstrate.

7. Process according to Claim 6, **characterized in that** acetaldehyde, propionaldehyde, butyraldehyde, valeraldehyde, capronaldehyde, benzaldehyde or o-phthalaldehdye is used as the aldehyde.

8. Process according to Claim 1, **characterized in that** compounds which contain an aryl-CHₙ (n = 1, 2 or 3) group or compounds which contain methylene or methine groups activated by carboxyl or carbonyl groups are used as a substrate.

9. Process according to Claim 1, **characterized in that** 0.001 to 0.7 mol of component a), 0.0001 mol to 0.5 mol of component b) and 0.1 to 0.3 mol of cosubstrate are used per mol of substrate.

10. Process according to Claim 1, **characterized in that** formic acid, acetic acid, propionic acid, acetonitrile, propionitrile, benzonitrile, formaldehyde, acetamide, dimethylformamide, dimethylacetamide, tert-butanol, tert-amyl alcohol, hexane, octane, benzene, chloroform, dichloromethane, dichloroethane, carbon tetrachloride, nitrobenzene, nitromethane, ethyl acetate, butyl acetate, dimethyl ether, diethyl ether, diisopropyl ether or mixtures thereof are used as solvents.

## Revendications

1. Procédé d'oxydation de substrats contenant des groupes méthyle (-CH₃), méthylène (=CH₂), ou méthyne (≡CH) au moyen d'une oxydation à l'oxygène moyennant l'utilisation d'un système de catalyseurs constitué d'un composé imide et d'un co-catalyseur métallique, **caractérisé en ce que** les substrats sont oxydés dans un solvant organique avec un aldéhyde aromatique ou aliphatique comprenant 2 à 20 atome de C, en présence d'un système de catalyseurs consistant en :
a) un composé imide de la formule 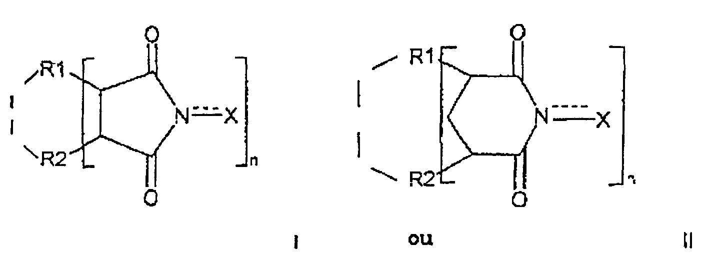 dans laquelle R₁ et R₂ peuvent signifier un H, un OH, un halogène, un groupe alkyle, alcényle ou alcoxyle en C₁ à C₂₀, un groupe aryle, un groupe acyle en C₁ à C₆, un groupe carboxyle ou un groupe alcoxycarbonyle en C₁ à C₁₀, ou R₁ et R₂ peuvent former ensemble dans la formule I une double liaison ou, dans la formule I ou II, un système cyclique aromatique ou non-aromatique, X peut signifier O ou OH et n peut être un nombre entier de 1 à 3, et
b) un co-catalyseur contenant un ou plusieurs éléments du groupe des métaux de transition et des éléments 2A et 3A du système périodique,
pour donner les composés oxo correspondants.

2. Procédé selon la revendication 1, **caractérisé en ce qu'**en tant que composé a) du système de catalyseurs est utilisé le N-hydroxy-succinimide, le N-hydroxy-maléimide, le N-hydroxy-hexahydrophtalimide, le N,N'-dihydroxy-cyclohexane tétracarboximide, le N-hydroxy-phtalimide, le N-hydroxy-tétrabromophtalimide, le N-hydroxy-tétrachlorophtalimide, le N-hydroxytrimellitimide, le N,N'-dihydroxy-naphtalène tétracarboximide, le N-hydroxy(dodécényl)succinimide, le N-hydroxy-(octényl)succinimide, le N-hydroxynaphtalimide.

3. Procédé selon la revendication 1, **caractérisé en ce que** des co-catalyseurs qui contiennent un ou plusieurs éléments des groupes 1B, 4B, 5B, 6B, 7B ou 8 du système périodique sont utilisés.

4. Procédé selon la revendication 3, **caractérisé en ce qu'**un co-catalyseur qui contient une combinaison des éléments des groupes 6B et 8 ou 1B est utilisé.

5. Procédé selon la revendication 1, **caractérisé en ce que** le co-catalyseur est utilisé comme formiate, acétate, propionate, acétylacétonate, naphténate, stéarate, nitrate, sulfate, phosphate, carbonate ou halogénure.

6. Procédé selon la revendication 1, **caractérisé en ce qu'**un aldéhyde aliphatique saturé ou insaturé, un aldéhyde aromatique ou un dialdéhyde avec 2 à 20 atomes de C est utilisé comme co-substrat.

7. Procédé selon la revendication 6, **caractérisé en ce qu'**en tant qu'aldéhyde est utilisé l'aldéhyde acétique, l'aldéhyde propionique, l'aldéhyde butyrique, l'aldéhyde valérique, l'aldéhyde caproïque, l'aldéhyde benzoïque ou l'aldéhyde o-phtalique.

8. Procédé selon la revendication 1, **caractérisé en ce qu'**en tant que substrat sont utilisés des composés contenant un groupe aryle-CHₙ (n = 1, 2 ou 3) ou des composés qui contiennent un groupe méthylène ou méthyne activé par un groupe carboxyle ou carbonyle.

9. Procédé selon la revendication 1, **caractérisé en ce que** par mole de substrat sont utilisés 0,001 à 0,7 mole du composant a), 0,0001 à 0,5 mole du composant b) et 0,1 à 0,3 mole de co-substrat.

10. Procédé selon la revendication 1, **caractérisé en ce qu'**en tant que solvant sont utilisés l'acide formique, l'acide acétique, l'acide propionique, l'acétonitrile, le propionitrile, le benzonitrile, l'aldéhyde formique, l'acétamide, le diméthyle formamide, le diméthyle acétamide, le t-butanol, l'alcool t-amylique, l'hexane, l'octane, le benzène, le chloroforme, le dichlorométhane, le dichloréthane, le tétrachlorure de carbone, le nitrobenzène, le nitrométhane, l'acétate d'éthyle, l'acétate de butyle, l'éther diméthylique, l'éther diéthylique, l'éther di-isopropylique ou des mélanges de ces corps.
